# EUROPÄISCHE PATENTANMELDUNG

(11) **EP 3 530 256 A1**
(43) Veröffentlichungstag der Anmeldung: **28.08.2019**
(21) Anmeldenummer: 18000176.0
(22) Anmeldetag: 21.02.2018
(51) Int. Cl.: A61J 7/00

(54) **TRINKHALM ZUR VERABREICHUNG EINES WIRKSTOFFS**

(71) Anmelder: DS-Technology GmbH, 71364 Winnenden (DE)
(72) Erfinder: Mössinger, Matthias, D - 71573 Allmersbach im Tal (DE); Sternberger-Rützel, Elke, D - 71573 Allmersbach im Tal (DE); Paetzold, Jan, D - 71573 Allmersbach im Tal (DE); Müller, Daniel, D - 71573 Allmersbach im Tal (DE)
(74) Vertreter: Schmid, Barbara

(57) **Zusammenfassung**

Die Erfindung betrifft einen Trinkhalm (10) zur Verabreichung eines Wirkstoffs (12). Der Trinkhalm (10) besitzt eine Wandung (20) mit einem ersten Ende und mit einem zweiten Ende. Eines der beiden Enden der Wandung (20) kann mit einem Verschlusselement (30) lösbar verschlossen werden. Im Bereich des anderen Endes der Wandung (20) ist eine Membran (40) vorhanden, auf der der Wirkstoff (12) aufliegt.

## Beschreibung

### TECHNISCHES GEBIET

Die vorliegende Erfindung betrifft einen Trinkhalm zur Verabreichung von Wirkstoffen wie beispielsweise von Nahrungsergänzungsmitteln oder von Medikamenten. Der zu verabreichende Wirkstoff befindet sich bereits in dem Trinkhalm und wird mit einer Flüssigkeit aus dem Trinkhalm in den Mund gesaugt und anschließend geschluckt. Dies erleichtert regelmäßig die Einnahme des Wirkstoffs, weil dieser sehr viel leichter geschluckt werden kann. Durch die Aufnahme des Wirkstoffs als in-situ-Suspension mit einer Flüssigkeit kann darüber hinaus der als unangenehm empfundene Geschmack des Wirkstoffs durch die Flüssigkeit überdeckt werden.

### STAND DER TECHNIK

Trinkhalme zur Verabreichung von Wirkstoffen sind beispielsweise aus der WO 98/51259 A1 oder der WO 2006/079648 A1 bekannt. Die dort beschriebenen Trinkhalme weisen eine Wandung mit einem ersten und mit einem zweiten Ende auf, wobei die Wandung an dem ersten und/ oder dem zweiten Ende eine Versteifung aufweisen kann. Im Inneren des Trinkhalms befindet sich ein Controller. Dieser Controller verhindert, dass der ebenfalls im Trinkhalm befindliche Wirkstoff unten aus dem Trinkhalm hinausfällt. Der Controller befindet sich ursprünglich eher im unteren Bereich des Trinkhalms. Während des Trinkens wird der Controller nach oben gesaugt und verbleibt auch nach dem Absetzen des Trinkhalms an dieser neuen Position. Durch die Position des Controllers kann dadurch auf einfache Weise kontrolliert werden, ob der Wirkstoff bereits vollständig eingenommen wurde oder nicht.

Die Controller sind in der Regel als offenporige Festkörper ausgebildet und aus einem gas- und/oder flüssigkeitsdurchlässigem Material. So können die Controller beispielsweise in Form eines Schwämmchens oder in Form eines Fasergeflechts ausgebildet sein.

Die Controller wurden dabei bislang für die Verabreichung von Wirkstoffen in Form von Pellets entwickelt. Die verwendeten Pellets weisen dabei eine bestimmte Mindestgröße und eine definierte Partikelgrößenverteilung auf. Bei der Verabreichung von Wirkstoffen in Form von feinen Pulvern kann es dagegen vorkommen, dass diese durch den Controller durchsickern und bereits vor dem Beginn des Saugvorgangs beispielsweise in die Verpackung des Trinkhalms austreten. Auch kann regelmäßig nicht gewährleistet werden, dass die feinen Pulver vollständig eingenommen werden, da ein Teil des feinen Pulvers in dem Controller hängen bleiben kann. Dies kann insbesondere bei der Verabreichung von potentiell toxischen Medikamenten - beispielsweise bei Onkologika - zu einer Gefährdung Dritter führen, da diese Dritte so versehentlich mit dem Wirkstoff in Kontakt kommen können. Die Verabreichung von Wirkstoffen in Form von feinen Pulvern mit einer Partikelgröße unter 210 Mikrometern ist daher mit den beschriebenen Controllern nicht möglich.

### DARSTELLUNG DER ERFINDUNG

Ausgehend von diesem vorbekannten Stand der Technik lag der Erfindung die Aufgabe zugrunde, einen Trinkhalm zur Verabreichung eines Wirkstoffs anzugeben, mit dem auch feinpulvrige Wirkstoffe sicher verabreicht werden können.

Der erfindungsgemäße Trinkhalm zur Verabreichung eines Wirkstoffs ist durch die Merkmale des Hauptanspruchs 1 gegeben. Sinnvolle Weiterbildungen der Erfindung sind Gegenstand von sich an diesen Anspruch anschließenden weiteren Ansprüchen.

Der erfindungsgemäße Trinkhalm zur Verabreichung eines Wirkstoffes besitzt eine Wandung mit einem ersten Ende und mit einem zweiten Ende und ein Verschlusselement, durch das eines der beiden Enden der Wandung lösbar verschlossen werden kann. Erfindungsgemäß ist im Bereich des anderen Endes der Wandung eine Membran vorhanden. Auf dieser Membran kann der Wirkstoff aufliegen, so dass der Wirkstoff zwischen der Membran und dem Verschlusselement im Innenraum der Wandung eingeschlossen ist. Die Verwendung einer Membran anstelle des Controllers ermöglicht es, auch feine Pulver mittels des erfindungsgemäßen Trinkhalms sicher und exakt dosiert verabreichen zu können.

In einer ersten Ausführungsform kann die Membran flüssigkeitsdurchlässig ausgebildet sein. Die Membran kann daher während des Trinkvorgangs intakt bleiben, da die Flüssigkeit, die zur Aufnahme des Wirkstoffs getrunken wird, durch die Membran gelangen kann. Auf diese Weise kann auch sichergestellt werden, dass keine unerwünschten Feststoffe versehentlich mit aufgenommen werden.

In einer zweiten Ausführungsform kann die Membran während des Saugvorgangs eine Öffnung bilden, durch die Flüssigkeit durch die Membran durchtreten kann. Die Membran kann in diesem Fall beispielsweise kontrolliert aufplatzen oder reißen, wodurch sich die entsprechende Öffnung bilden kann. Um die Bildung der Öffnung kontrolliert ablaufen zu lassen, kann die Membran vorzugsweise eine Sollbruchstelle aufweisen, die insbesondere als Materialverdünnung ausgebildet sein kann. Eine solche Sollbruchstelle in Form einer Materialverdünnung kann beispielsweise durch das Abtragen von Material mittels eines Lasers erfolgen. Die Sollbruchstelle sollte sich insbesondere im mittleren Bereich der Membran befinden und nicht an deren Randbereichen. Auf diese Weise kann verhindert werden, dass sich die Membran ganz oder teilweise von der Wandung des Trinkhalms löst.

Unabhängig von der Ausgestaltung der Membran kann die Membran in einer ersten Ausführungsform an der Stirnseite der Wandung des Trinkhalms befestigt sein. Eine solche Befestigung kann beispielsweise über ein geeignetes Klebemittel oder durch Aufsiegeln der Membran auf die Stirnseite erfolgen.

Um eine nachträgliche Manipulation der Membran nach der Befüllung des Trinkhalms mit dem Wirkstoff zu vermeiden, kann die Membran in einer bevorzugten Ausführungsform ein Stück nach innen versetzt angeordnet und somit an der Innenwandung des Trinkhalms befestigt sein.

In einer konstruktiv besonders einfachen Ausführungsform kann die Membran mittels eines Klemmelements an der Innenwandung des Trinkhalms befestigt sein. Ein solches Klemmelement kann insbesondere einen Rohrstutzen besitzen, dessen kreisförmiger Außendurchmesser geringfügig kleiner ist als der kreisförmige Innendurchmesser der Wandung des Trinkhalm. Wird ein solcher Rohrstutzen mit einem durchgehenden Schlitz in Längsrichtung versehen, dehnt sich der Rohrstutzen aus, so dass sich dessen Außendurchmesser vergrößert. Der Rohrstutzen kann in diesem Fall durch lediglich geringe Kraftaufwendung zusammengedrückt werden und auf diese Weise ein Stück weit in den Innenraum der Wandung eingeführt werden. Nach der Positionierung des Rohrstutzens kann sich dieser ausdehnen und sich dicht an die Innenwand des Trinkhalms pressen. Dadurch kann eine Membran, die über den Rohrstutzen gelegt wurde, sicher innerhalb des Trinkhalms fixiert werden. Die Membran kann im einfachsten Fall lose über dem Rohrstutzen des Klemmelements liegen. Alternativ dazu kann die Membran auch beispielsweise mittels eines Klebemittels an dem Rohrstutzen befestigt sein. Es wäre auch möglich, die Membran auf dem Rohrstutzen aufzusiegeln.

Um zu verhindern, dass der Wirkstoff während der Lagerung oder des Transports aus dem der Membran gegenüber liegenden Ende der Wandung aus dem Trinkhalm herausrieselt, ist das entsprechende Ende der Wandung mittels eines Verschlusselements verschlossen. Das Verschlusselement kann unmittelbar vor der Einnahme des Wirkstoffs von dem Patienten oder einem Helfer entfernt werden, so dass dieses Ende des Trinkhalms von dem Patienten in den Mund genommen werden kann. Das Verschlusselement kann im einfachsten Fall eine Kappe sein, die reibschlüssig über das Ende der Wandung gestülpt werden kann. Um ein Herausrieseln der feinen Pulver möglichst effektiv zu verhindern, kann das Verschlusselement vorzugsweise als Folie ausgebildet sein. Die Folie kann dabei lösbar an der Stirnseite der Wandung befestigt sein. Eine solche Befestigung kann beispielsweise über ein geeignetes Klebemittel oder durch ein Aufsiegeln der Folie erfolgen. Alternativ dazu kann das Verschlusselement lösbar an der Außenwandung des Trinkhalms befestigt sein. Dies kann beispielsweise durch eine Schrumpffolie, insbesondere durch einen Schrumpfschlauch oder eine Schrumpfkappe, als Verschlusselement realisiert werden. Das Verschlusselement kann darüber hinaus mit einem zusätzlichen Klebeband an der Außenwandung des Trinkhalms fixiert werden.

Um die Membran zu schützen, kann das membranseitige Ende der Wandung durch eine Sperrschicht verschlossen sein. Die Sperrschicht sollte luftdicht sein, so dass nicht versehentlich Luft durch den Trinkhalm eingesaugt werden kann. Dies könnte dazu führen, dass die Membran bereits durch ein Ansaugen von Luft versehentlich reißen könnte. In diesem Fall besteht die Gefahr, dass der Patient den feinpulvrigen Wirkstoff einatmet. Gleichzeitig sollte die Sperrschicht wasserlöslich sein, so dass sich diese beim Kontakt mit einem Getränk auflöst. Auf diese Weise kann nach kurzzeitigem Stehen des Trinkhalms in einem Getränk die volle Funktionsfähigkeit des Trinkhalms gewährleistet werden. Nach dem Auflösen der Sperrschicht kann das Getränk durch den Trinkhalm aufgesaugt werden, wodurch auch die Aufnahme des Wirkstoffs ermöglicht wird. Die Sperrschicht kann insbesondere aus einem geschmacksneutralem Polymer, beispielsweise aus Gelatine, bestehen.

Die Wandung des erfindungsgemäßen Trinkhalms kann an dem ersten und/oder an dem zweiten Ende eine Versteifung aufweisen, wie sie beispielsweise aus der WO 2006/079648 A1 bereits bekannt ist.

Ein Trinkhalm im Sinne dieser Erfindung ist jeder Trinkhalm, mit dem durch den Mund eines Menschen eine Flüssigkeit eingesaugt werden kann.

Der erfindungsgemäße Trinkhalm kann vorzugsweise aus einem thermoplastischen oder duroplastischen Kunststoff bestehen. Sofern der Inhalt des Trinkhalms sichtbar sein soll, kann die Wandung des Trinkhalms aus einem transparenten Material, insbesondere aus einem farblosen transparenten Material bestehen. Auf diese Weise kann sofort gesehen werden, ob sich der Wirkstoff noch in dem Trinkhalm befindet, oder ob dieser bereits eingenommen wurde.

Weitere Vorteile und Merkmale der Erfindung sind den in den Ansprüchen ferner angegebenen Merkmalen sowie den nachstehenden Ausführungsbeispielen zu entnehmen.

### KURZE BESCHREIBUNG DER ZEICHNUNG

Die Erfindung wird im Folgenden anhand der in der Zeichnung dargestellten Ausführungsbeispiele näher beschrieben und erläutert. Es zeigen:
- Fig. 1: einen Längsschnitt durch eine erste Ausführungsform des erfindungsgemäßen Trinkhalms vor dem Beginn des Aufsaugens des Wirkstoffs,
- Fig. 2: einen Längsschnitt durch den Trinkhalm gemäß Fig 1 nach dem Aufsaugen des Wirkstoffs,
- Fig. 3: einen Querschnitt durch den Trinkhalm gemäß Fig. 1 entlang der Linie A-A,
- Fig. 4: einen Längsschnitt durch eine zweite Ausführungsform des erfindungsgemäßen Trinkhalms vor dem Beginn des Aufsaugens des Wirkstoffs,
- Fig. 5: einen Längsschnitt durch den Trinkhalm gemäß Fig. 4 nach dem Beginn des Aufsaugens des Wirkstoffs,
- Fig. 6: eine Draufsicht auf die intakte Membran des Trinkhalms gemäß Fig. 4,
- Fig. 7: eine Draufsicht auf die aufgerissene Membran des Trinkhalms gemäß Fig. 5
- Fig. 8: einen Längsschnitt durch eine dritte Ausführungsform des erfindungsgemäßen Trinkhalms vor dem Beginn des Aufsaugens des Wirkstoffs,
- Fig. 9: einen Längsschnitt durch den Trinkhalm gemäß Fig. 8 nach dem Beginn des Aufsaugens des Wirkstoffs,
- Fig. 10: eine Draufsicht auf die intakte Membran des Trinkhalms gemäß Fig. 8
- Fig. 11: eine Draufsicht auf die aufgerissene Membran des Trinkhalms gemäß Fig 11
- Fig. 12: eine perspektivische Ansicht des oberen Endes des Trinkhalms, der mit einer aufgesiegelten Folie verschlossen ist, und
- Fig. 13: eine perspektivische Ansicht des oberen Endes des Trinkhalms, der mit einer Schrumpfhaube verschlossen ist.

### WEGE ZUM AUSFÜHREN DER ERFINDUNG

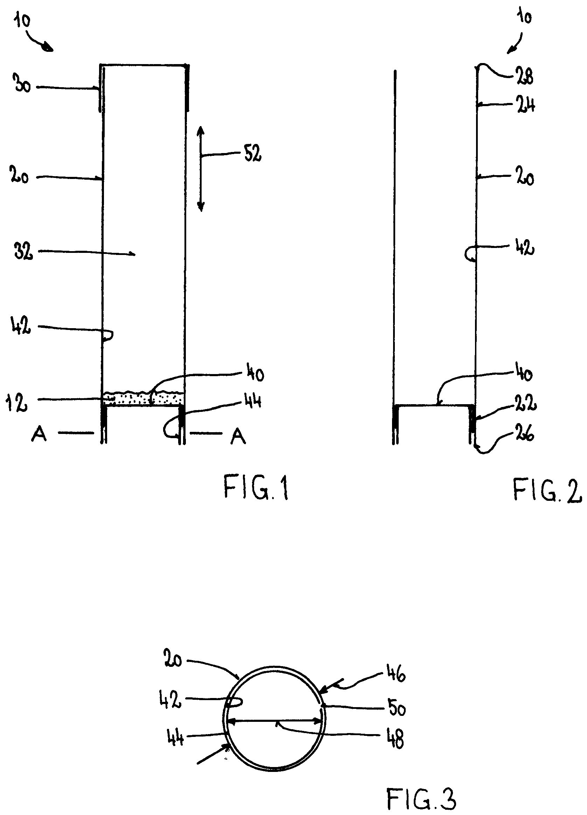

In den Fig. 1 bis 3 ist eine erste Ausführungsform des erfindungsgemäßen Trinkhalms 10 zur Verabreichung eines Wirkstoffs 12 dargestellt. Der Trinkhalm 10 besitzt eine Wandung 20 mit einem ersten, unteren Ende 22 und einem zweiten, oberen Ende 24. Das untere Ende 22 kann in ein Gefäß mit einer trinkbaren Flüssigkeit gestellt werden. Das obere Ende 24 wird in den Mund genommen, so dass die Flüssigkeit durch den Trinkhalm 10 aufgesaugt werden kann.

Das obere Ende 24 der Wandung 20 ist vor dem Gebrauch durch ein Verschlusselement in Form einer Kappe 30 verschlossen. Die Kappe 30 sitzt reibschlüssig auf dem oberen Ende 24 der Wandung 20 auf. Dadurch kann der im Innenraum 32 der Wandung 20 befindliche Wirkstoff nicht oben aus dem Trinkhalm 10 herausrieseln.

Im Bereich des unteren Endes 22 der Wandung 20 ist eine Membran 40 vorhanden. Die Membran 40 ist ausreichend dicht ausgebildet, so dass auch feinpulvrige Wirkstoffe 12 nicht durch die Membran 40 durchrieseln können. Gleichzeitig ist die Membran 40 so glatt ausgebildet, dass die Wirkstoffe 12 nicht an der Membran 40 anhaften können. Die Membran 40 ermöglicht daher die Verabreichung von feinpulvrigen, wirkstoffhaltigen Formulierungen durch den erfindungsgemäßen Trinkhalm 10. Die wirkstoffhaltigen Formulierungen können dabei einen einzelnen Wirkstoff oder auch eine Mischung verschiedener Wirkstoffe enthalten. Die Formulierungen können darüber hinaus in beiden Fällen neben den Wirkstoffen noch zusätzliche Füllstoffe enthalten.

Die Membran 40 ist im vorliegenden Beispielsfall an der Innenwandung 42 des Trinkhalms 10 befestigt. Die Membran 40 ist dabei über ein Klemmelement in Form eines Rohrstutzens 44 gespannt. Der Rohrstutzen 44 besitzt einen Außendurchmesser 46, der etwas kleiner ist als der Innendurchmesser 48 der Wandung 20 des Trinkhalms 10. In dem Rohrstutzen 44 ist ein durchgehender Schlitz 50 in Längsrichtung 52 ausgebildet. Durch diesen Schlitz 50 kann sich der Rohrstutzen 44 ein Stück weit ausdehnen, so dass dieser dicht an der Innenseite 42 der Wandung 20 anliegt. Die zwischen der Außenseite des Rohrstutzens 44 und der Innenseite 42 der Wandung 20 befindliche Membran 40 wird somit an der gewünschten Position fixiert.

Auf der Membran 40 kann der zu verabreichende Wirkstoff 12 aufliegen. Der Wirkstoff 12 befindet sich dann zwischen der Membran 40 und der Kappe 30 im Innenraum des Trinkhalms 10 (siehe Fig. 1). Zum Transport und zur Lagerung des Trinkhalms 10 kann dieser zusätzlich in einer hier nicht dargestellten Umverpackung, beispielsweise in einem Folienbeutel, verpackt sein.

Die Membran 40 ist in dem Ausführungsbeispiel gemäß Fig. 1 bis 3 flüssigkeitsdurchlässig ausgebildet. Die zu trinkende Flüssigkeit kann somit durch die Membran 40 hindurchgesaugt werden. Dabei kann der zu verabreichende Wirkstoff 12 in der Flüssigkeit gelöst oder suspendiert werden und auf diese Weise in den Mund es Patienten gelangen. Die Membran 40 bleibt dabei unversehrt und liegt nach dem Ende des Trinkvorgangs (siehe Fig. 2) unverändert vor.

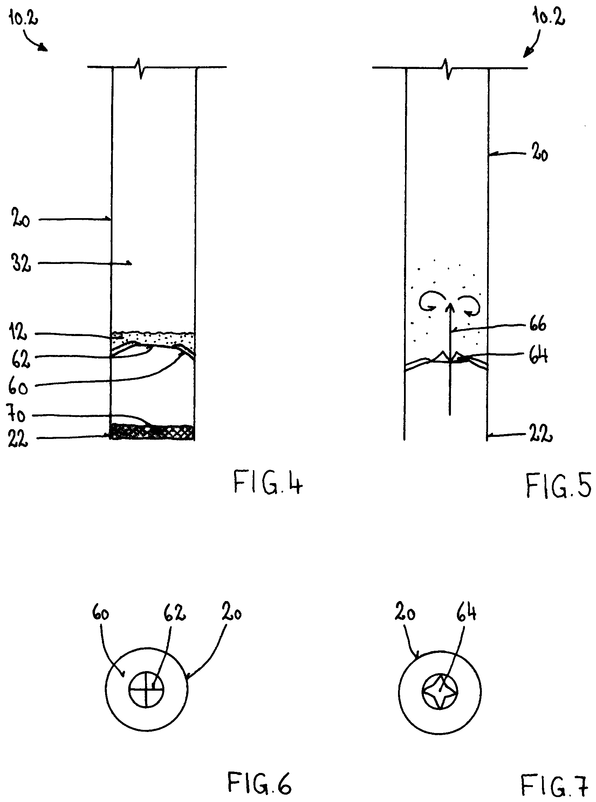

In den Fig. 4 bis 7 ist eine zweite Ausführungsform des erfindungsgemäßen Trinkhalms 10.2 dargestellt. Die Membran 60 ist in diesem Fall an der Innenwandung 42 des Trinkhalms 10.2 angeschweißt. Die Membran 60 besitzt eine Sollbruchstelle in Form einer Materialverdünnung 62. Die Materialverdünnung 62 kann beispielsweise mittels eines Lasers hergestellt werden. Durch einen Laser können einzelne Schichten der Membran 60 abgetragen werden, so dass diese zwar nach wie vor als Abdichtung für den zu verabreichenden Wirkstoff 12 dienen kann, jedoch bei einer Druck- oder Saugbelastung einreißt.

Die Materialverdünnung 62 ist im vorliegenden Beispielsfall im mittleren Bereich der Membran 60 vorgesehen. Die Membran 60 reißt dadurch mittig auf, bleibt jedoch randseitig sicher an der Innenwandung 42 des Trinkhalms 10.2 befestigt. Dadurch kann sichergestellt werden, dass die Membran 60 nicht versehentlich so reißt, dass Teile der Membran 60 mit eingesaugt werden können. Die Materialverdünnung 62 ist im vorliegenden Beispielsfall kreuzförmig ausgeführt (siehe Fig. 6), so dass sich eine flächige Öffnung 64 (siehe Fig. 7) bildet, sobald die Membran 60 einreißt. Durch diese Öffnung 64 kann die Flüssigkeit aufgesaugt werden (Pfeil 66). Dabei wird der zu verabreichende Wirkstoff 12 mitgerissen und kann ebenfalls aufgesaugt und geschluckt werden.

Die Membran 60 ist wie die Membran 40 auch ausreichend dicht ausgebildet, so dass auch feinpulvrige Wirkstoffe 12 nicht durch die Membran 60 durchrieseln können. Gleichzeitig ist die Membran 60 so glatt ausgebildet, dass die Wirkstoffe 12 nicht an der Membran 60 anhaften können.

Um ein vorzeitiges Reißen der Membran 60 zu verhindern, ist an dem membranseitigen unteren Ende 22 der Wandung 20 im vorliegenden Ausführungsbeispiel eine Sperrschicht 70 vorgesehen. Die Sperrschicht 70 schließt den Innenraum 32 des Trinkhalms 10 nach unten hin luftdicht ab. Auf diese Weise kann verhindert werden, dass die Membran 60 bereits beim Ansaugen von Luft reißt und dadurch der Wirkstoff 12 versehentlich eingeatmet werden könnte. Die Sperrschicht 70 löst sich nach kurzer Zeit des Stehens in einem Getränk auf. Dann kann das Getränk durch den Trinkhalm 10 aufgesaugt werden. Erst dabei reißt die Membran 60 ein und der Wirkstoff 12 kann aufgenommen und geschluckt werden.

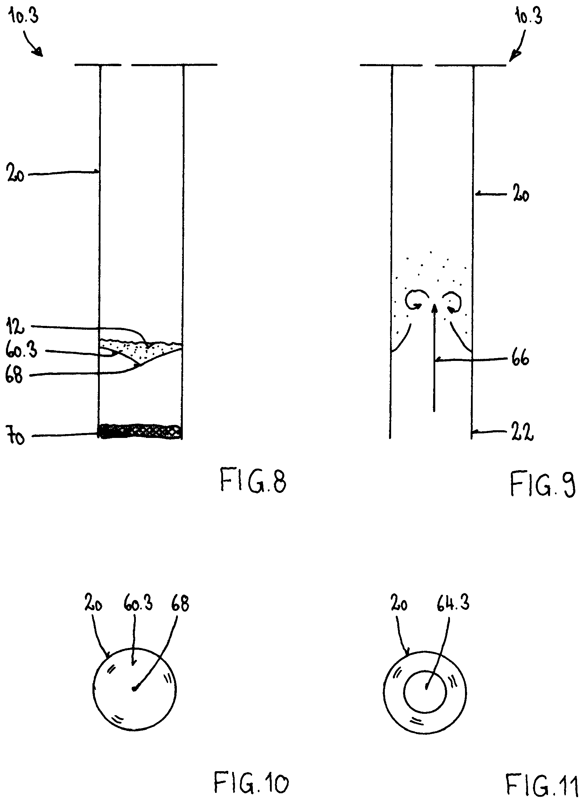

In den Fig. 8 bis 11 ist eine dritte Ausführungsform des erfindungsgemäßen Trinkhalms 10.3 dargestellt. Die Membran 60.3 ist auch in diesem Fall an der Innenwandung 42 des Trinkhalms 10.3 befestigt. Die Membran 60.3 besitzt eine Sollbruchstelle in Form einer Laserperforation 68 (siehe Fig. 10). Bei einer Druck- oder Saugbelastung reißt die Laserperforation 68 auf und es bildet sich im mittleren Bereich der Membran 60.3 eine kreisförmige Öffnung 64.3 (siehe Fig. 11). Durch diese Öffnung 64.3 kann die Flüssigkeit aufgesaugt werden (Pfeil 66). Dabei wird der zu verabreichende Wirkstoff 12 mitgerissen und kann ebenfalls aufgesaugt und geschluckt werden. Auch bei dieser Ausführungsform ist an dem unteren Ende 22 der Wandung 20 zunächst die Sperrschicht 70 vorgesehen (siehe Fig 10).

Die Sperrschicht 70 könnte auch bei dem ersten Ausführungsbeispiel gemäß Fig. 1 bis 4 vorgesehen werden.

Im Gegensatz zu den hier dargestellten Ausführungsbeispielen könnte die Membran 40, 60, 60.3 auch an der Stirnseite 26 des unteren Endes 22 der Wandung 20 befestigt sein.

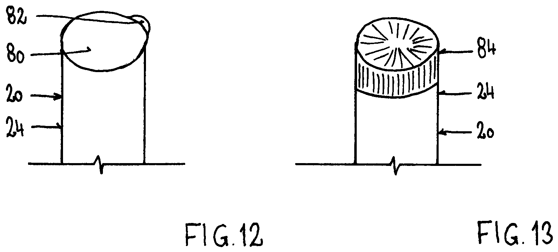

In Fig. 12 und 13 sind zwei alternative Verschlusselemente für das obere Ende 24 der Wandung 20 dargestellt. Gemäß Fig. 12 ist das Verschlusselement als Siegelfolie 80 ausgebildet. Die Siegelfolie 80 ist lösbar an der Stirnseite 28 des oberen Endes 24 der Wandung 20 befestigt. Die Siegelfolie 80 weist dazu eine Lasche 82 auf. Mittels der Lasche 82 kann die Siegelfolie 80 von der Stirnseite 28 abgezogen werden, um den Wirkstoff 12 aufsaugen zu können.

Gemäß Fig. 13 ist das Verschlusselement als Schrumpfkappe 84 ausgebildet. Die Schrumpfkappe 84 kann von der Wandung 20 abgezogen werden, um das obere Ende 22 der Wandung 20 freizulegen. Alternativ dazu könnte die Schrumpfkappe 84 auch eine oder mehrere Perforationen aufweisen, die ein Aufreißen und damit Entfernen der Schrumpfkappe 84 erleichtern können. Anstelle der Schrumpfkappe 84 könnte beispielsweise auch ein Schrumpfschlauch verwendet werden.

## Patentansprüche

1. Trinkhalm (10, 10.2, 10.3) zur Verabreichung eines Wirkstoffs (12),
- mit einer Wandung (20) mit einem ersten Ende (22) und mit einem zweiten Ende (24),
- mit einem Verschlusselement (30, 70, 80), durch das eines der beiden Enden (24) der Wandung (20) lösbar verschließbar ist,
- **dadurch gekennzeichnet, dass**
- im Bereich des anderen Endes (22) der Wandung (20) eine Membran (40, 60, 60.3) vorhanden ist,
- der Wirkstoff (12) auf der Membran (40, 60, 60.3) aufliegt.

2. Trinkhalm nach Anspruch 1,
- **dadurch gekennzeichnet, dass**
- die Membran (40) flüssigkeitsdurchlässig ausgebildet ist.

3. Trinkhalm nach Anspruch 1 oder 2,
- **dadurch gekennzeichnet, dass**
- im Bereich der Membran (60, 60.3) eine Öffnung (64, 64.3) zum Einsaugen von Flüssigkeit bildbar ist.

4. Trinkhalm nach Anspruch 3,
- **dadurch gekennzeichnet, dass**
- die Membran (60, 60.3) zumindest eine Sollbruchstelle (62, 68) aufweist, die insbesondere als Materialverdünnung (62) ausgebildet ist.

5. Trinkhalm nach einem der vorstehenden Ansprüche,
- **dadurch gekennzeichnet, dass**
- die Membran (40, 60, 60.3) an der Innenwandung (42) des Trinkhalms (10, 10.2, 10.3) befestigt ist.

6. Trinkhalm nach Anspruch 5,
- **dadurch gekennzeichnet, dass**
- die Membran (40) mittels eines Klemmelements (44) an der Innenwandung (42) des Trinkhalms (10) befestigt ist.

7. Trinkhalm nach Anspruch 6,
- **dadurch gekennzeichnet, dass**
- das Klemmelement einen Rohrstutzen (44) besitzt, dessen Außendurchmesser (46) geringfügig kleiner ist als der Innendurchmesser (48) der Wandung (20) des Trinkhalms (10),
- der Rohrstutzen (44) einen durchgehenden Schlitz (50) in Längsrichtung (52) aufweist.

8. Trinkhalm nach einem der vorstehenden Ansprüche,
- **dadurch gekennzeichnet, dass**
- das Verschlusselement als Folie (80, 84) ausgebildet ist.

9. Trinkhalm nach Anspruch 8,
- **dadurch gekennzeichnet, dass**
- das Verschlusselement (80) an der Stirnseite (28) der Wandung (20) lösbar befestigt ist, insbesondere angeklebt oder angeschweißt ist.

10. Trinkhalm nach Anspruch 8 oder 9,
- **dadurch gekennzeichnet, dass**
- das Verschlusselement (84) an der Außenwandung des Trinkhalms (10.3) lösbar befestigt ist.

11. Trinkhalm nach Anspruch 10,
- **dadurch gekennzeichnet, dass**
- das Verschlusselement als Schrumpffolie, insbesondere als Schrumpfkappe (84) oder als Schrumpfschlauch, ausgebildet ist.

12. Trinkhalm nach einem der vorstehenden Ansprüche,
- **dadurch gekennzeichnet, dass**
- das membranseitige Ende (22) der Wandung (20) durch eine wasserlösliche Sperrschicht (70) luftdicht verschlossen ist.

13. Trinkhalm nach Anspruch 12,
- **dadurch gekennzeichnet, dass**
- die wasserlösliche Sperrschicht (70) aus einem Polymer, insbesondere aus Gelatine, besteht.
